# EUROPEAN PATENT SPECIFICATION

(11) **EP 1 210 601 B1**
(45) Date of publication and mention of the grant of the patent: **12.07.2006**
(21) Application number: 00963208.4
(22) Date of filing: 07.09.2000
(51) Int. Cl.: G01N 33/543

(54) **SELECTIVELY BINDING AND MAGNETICALLY RESPONSIBLE NANOPARTICLES**
SELEKTIV BINDENDE UND MAGNETISCHE NANOPARTIKEL
NANOPARTICULES A LIAISON SELECTIVE ET A REPONSE MAGNETIQUE

(30) Priority: 08.09.1999 SE 9903183
(43) Date of publication of application: 05.06.2002
(73) Proprietor: European Institute of Science AB, 223 70 Lund (SE)
(72) Inventor: FREDRIKSSON, Sarah, S-245 61 Staffanstorp (SE); KRIZ, Dario, S-243 95 Höör (SE)
(74) Representative: Bjerre, Nils Birger Johannes
(86) International application number: PCT/SE2000/001729
(87) International publication number: WO 2001/022088

(56) References cited:
- WO-A1-97/35200
- WO-A2-94/21240
- US-A- 5 492 814

## Description

The present invention relates to magnetically responsible particles which are applicable for medical use.

### BACKGROUND OF THE INVENTION

The main component in commercially available magnetically responsible particles in most cases consists of ferrite/magnetite (a special type of iron oxide having magnetic properties, i.e. its relative magnetic permeability is very high). The particles contain a superparamagnetic core which can be enclosed in or mixed with a polymer, such as dextran or protein, or surrounded by an outer monolayer or bilayer of amphiphatic molecules, such as fatty acids or derivates thereof. This outer coating consisting of polymer or amphiphatic molecules prevents the superparamagnetic core from aggregating with other adjacent cores and forming larger particles which have undesired permanent-magnetic properties (1). The outer coating has been used for chemical association of other molecules, for instance, receptors and antibodies, to the surface of the magnetically responsible particles, which thus obtain selectively binding properties.

Presently, magnetically responsible particles, in the size of ten nanometres up to a few micrometres, with modified surfaces are commercially available for various purposes, such as preparation of RNA, synthesis of cDNA libraries on solid phase, protein purification, carriers in immunologic analyses, ion exchange and affinity chromatography and purification of cells, viruses, organelles and proteins (2, 3). Such particles have also recently proved to be usable as markers in immunologic analyses (1).

Development of magnetically responsible particles in the size of one to a few hundred nanometres is in progress, since such particles are sufficiently small to form stable colloidal aqueous solutions, so-called ferrofluids which can be used for injection in humans and animals. A suitably constructed particle with optimal properties as to selectivity, biocompatibility and toxicity offers the possibility of recognising and binding to different targets (such as tissues or organs) in an organism, which enables the development of new powerful drugs against e.g. cancer.

It is already known that the advantage of using magnetically responsible particles in connection with therapy is that toxicity can be produced directly (heating of the core) or indirectly (thermal activation via the core of thermoactivatable toxic molecules which are bound to the coating) when the particles are exposed to an applied magnetic field.

By associating special recognition molecules to the coating of the magnetically responsible particle, the particles can be concentrated in vivo in a target. The type of molecules which are capable of specifically recognising and binding to other biomolecules can, for instance, be a receptor, a receptor ligand, an enzyme or an antibody. In the applications described in the literature, use is primarily made of antibodies which are directly bound to the magnetically responsible particles to be able to target said particles (4, 5).

However, when the size of the magnetically responsible particles is in the nanometre range (one to a few hundred nanometres) there may be problems in associating one or more native proteins to the limited surface without getting undesirable intermolecular interactions (e.g. steric blocking of active sites) or an undesirable increase of the particle size (destabilises the colloidal properties, undesirable aggregation and obstructed passage through biological structures and membranes).

To ensure the provision of the colloidal stability of ferrofluids, the particle size of the recognition molecule should not exceed 10 nm, whereas a protein has an average size of 2-10 nm (6).

### SUMMARY OF THE INVENTION

The invention relates to a magnetically responsible particle comprising a superparamagnetic core and an outer coating which contains at least one type of synthetic or partially synthetic peptide having at least one recognition function.

Preferably, the particle has a diameter of 10 nm or less.

At least one of the constituents included in the core suitably consists of at least one metal or a derivative thereof, such as an oxide.

In one embodiment of the particle, the core and the coating do not form separate units but constitute a chemically homogeneous mass.

The synthetic or partially synthetic peptide consist of a peptide having a length of 1 to 30 amino acids.

The particle according to the present invention can be used as a therapeutic agent in combination with an alternating homogeneous magnetic field or gradient magnetic field for treating different types of diseases. Examples of diseases are cancer, fungal and bacterial infections, protozoan infections, virus infections, metabolic disorders, immune defence diseases and genetically related diseases.

The therapeutic effect of the particle can be further enhanced by modifying its surface with one or more effector molecules comprising, inter alia, cytostatics, toxins, enzymes, heavy metals, biocompatibility promoting substances, substances which facilitate the transport of the particle through vascular walls and tissues.

The particle according to the invention can also be used as a subcomponent in bioassay.

### BRIEF DESCRIPTION OF THE DRAWING

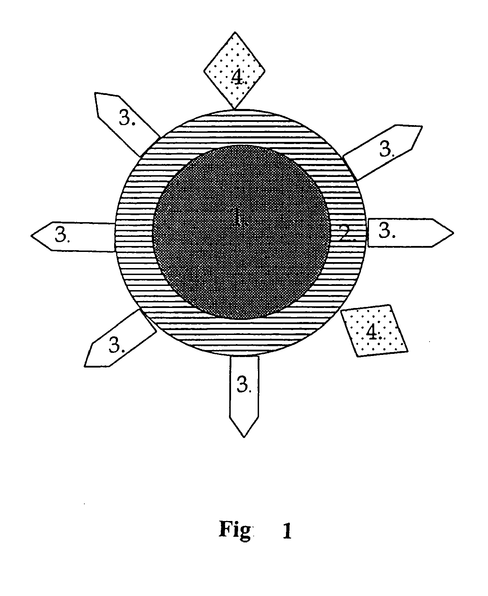

Fig. 1 is a schematic drawing of the particle according to the present invention.

### DETAILED DESCRIPTION OF THE INVENTION

The particle according to the invention utilises synthetic peptides instead of proteins to circumvent the above-mentioned problems. By starting from short (1-30 amino acids which corresponds to 1-10 % of a native protein) synthetic or partially synthetic peptides, which have been selected by computer design or from a synthetic peptide library using the target cell or the target molecule as a reference, the problems when using native proteins are circumvented.

Further advantages of a synthetic or semisynthetic peptide is the increase in stability for which these peptides can also be selected, because the binding activity of the recognition molecule is not as sensitive during production and handling as, for instance, an antibody.

The synthetic or partially synthetic peptides can be replaced by selectively binding synthetic or partially synthetic carbohydrate-based polymers, which consist of 1-30 identical or different carbohydrate units.

Patent Specification US 4,323,056 (Borelli et al) (7) discloses ferromagnetic particles designed for cancer therapy. These particles contain a magnetically responsible core incorporated in a ceramic material, which is derivatised with tumour-specific ions, antibodies and/or similar bioactive molecules directed towards the tumour. However, in the present invention use is not at all made of native proteins or defined bioactive molecules, which makes the particles described herein unique.

The magnetically responsible particle and a possible configuration are illustrated in Fig. 1. The magnetically responsible core 1 in the particle mainly consists of magnetite (iron oxide). Furthermore, the particle is covered with an outer layer 2 consisting of a derivatised polymer (Dextran) or a monolayer or alternatively a bilayer of derivatised fatty acids. The choice of the type (number of amino acid or carbohydrate units and sequence) of ligand 3 to be used for the derivatisation is individually adjusted for each application of the magnetically responsible particle, whose therapeutic effect can be further enhanced by further modifying its surface with one or more effector molecules 4.

The manufacturing process of magnetically responsible particles containing a synthetic polypetide with recognition function for HIV virus is described in Example I. Naturally, it is not difficult for the one skilled in the art to modify the formula by using alternative libraries or computer designs for the construction of the polypeptide, or alternatively to choose other targets than HIV virus, such as cells, bacteria, viruses, fungi, protozoa. Other alternatives consist in replacing the polypeptide by other artificial recognition molecules or using other metal salts, alternatively replacing Dextran by other polymers or fatty acids. It is, of course, also possible to replace the NaIO₄-coupling method by other prior-art coupling techniques.

The invention will be described in more detail by means of the following non-limiting Example.

### EXAMPLE 1

### Manufacture of magnetically responsible particles with affinity to HIV virus.

HIV virus protein capsids are adsorbed according to standard procedure protocol (8) (which is provided by the manufacturer) to wells in microtitre plates. Subsequently, 10¹¹ colony forming units (cfu) of an M13-phage library are added, with a DNA fragment inserted in the pIII gene of 36 randomised base pairs, in TBS with 1 mg/ml of bovine serum albumin. After 1 h incubation at 37°C, the wells are thoroughly washed with TBS-containing 1 mg/ml of bovine serum albumin. Selected bacteriophages are eluated by adding a solution with pH 2.2 containing 1 mg/ml of bovine serum albumin and 0.1 N HCl. The eluate is permitted to infect E. coli cells according to standard procedure protocols. Selected clones are DNA sequenced by means of standard procedure protocols, after which 100 µg of synthetic polypeptide with affinity to HIV virus is produced preparatively.

A solution containing 100 mM FeCl₂, 150 mM FeCl₃ and 6% Dextran (having a molecular weight of 70,000 g/mol) is precipitated in a beaker by adding 28% NH₃. The precipitation takes place at 50°C and is allowed to continue for 3 h. Subsequently, the solution is centrifuged (5500 rpm) for 50 min and finally dialysed against distilled water with a dialysis tubing (MWCO 70 kDa). The final product is a brownish solution containing magnetically responsible particles with an approximate diameter of 10 nm.

1 mg NaIO₄ is added to 1 ml of the brownish solution containing the magnetically responsible particles. The solution is allowed to stand for 20 min at 75°C, whereby the hydroxyl groups on the Dextran coating of the particles are activated. The synthetic polypeptide is added and the mixture is incubated over night, amino groups on the synthetic polypeptide binding covalently to the activated hydroxyl groups on the Dextran coating of the particles. Then blocking is performed with 10 mM glycine over night. The reaction mixture is dialysed against distilled water and evaporated, which finally results in a brown powder containing magnetically responsible particles (10 nm) having affinity to HIV virus.

### REFERENCES

1. Larsson, K. Kriz, K. and Kriz, D. Magnetic transducers in Biosensors and Bioassays. Analysis, No. 7, 1999.
2. Jordan, A., Wust P., Scholz R., Faehling H., Krause J. & Felix R. Magnetic Fluid Hyperthermia, 569-597, in Scientific and Clinical Applications of Magnetic Carriers, edited by Häfeli U., Schutt W., Teller J. and Zborowski M. Plenum Press 1997.
3. Mosbach, K. and Kriz, D. Framställning and användning av magnetiskt påverkbara polymerpartiklar. Swedish Patent Application SE 9400938-8.
4. Sestier C., Da Silva M.F., Sabolovic D., Roger J. & Pons J.N. Surface modification of superparamagnetic nanoparticles (Ferrofluid) studied with particle electrophoresis: Application to the specific targeting of cells. Electrophoresis, 19, 1220-1226, 1998.
5. Halbreich A., Roger J., Pons J.N., Geldwerth D., Da Silva M.F., Roudier M. & Bacri J.C. Biomedical applications of maghemite ferrofluid. Biochimie, 80, 379-90, 1998.
6. Mathews C.K., & van Holde K.E., BIOCHEMISTRY, The Benjamin/Cummings Publishing Company, 1990.
7. Borelli N.F., Luderer A.A. & Panzarino J. N. Radio frequency induced hyperthermia for tumor therapy. US Patent No. 4,323,056, 1982.
8. Scott J.K. & Smith G.P. *Biopanning.* Science, 249, 386-390, 1990.

## Claims

1. A magnetically responsible particle, **characterised in that** it comprises a superparamagnetic core and an outer coating which contains at least one type of synthetic non-native peptide with at least one recognition element, consisting of 1 to 30 amino acids, wherein said peptide has been selected by computer design or from a synthetic peptide library using a target cell or a target molecule.

2. A particle as claimed in claim 1, **characterised in that** its diameter is 10 nm or less.

3. A particle as claimed in claim 1 or 2, **characterised in that** at lest one of the constituents included in the core consists of at least one metal or a derivative thereof, such as an oxide.

4. A particle as claimed in any of claims 1-3, **characterised in that** the core and the coating do not form separate units but constitute a chemically homogeneous mass.

5. A particle as claimed in any of claims 1-4 for use as a therapeutic agent in combination with an alternating homogenous magnetic field or gradient magnetic field for treating different types of diseases.

6. A particle as claimed in claim 5 intended for treating cancer, fungal and bacterial infections, protozoan infections, virus infections, metabolic disorders, immune defence diseases and genetically related diseases.

7. A particle as claimed in claim 5 or 6, **characterised in that** the therapeutic effect of the particle has been further enhanced by modifying its surface with one or more effector molecules comprising, inter alia, cytostatics, toxins, enzymes, heavy metals, biocompatibility promoting substances, substances which facilitate the transport of the particle through vascular walls and tissues.

8. A particle as claimed in any of claims 1-4, for use as a subcomponent in bioassay.

## Patentansprüche

1. Magnetisch ansprechendes Teilchen, **dadurch gekennzeichnet, dass** es einen superparamagnetischen Kern und eine äußere Beschichtung umrasst, welche mindestens eine Art eines synthetischen, nicht nativen Peptids mit mindestens einem Erkennungselement enthält, das aus 1 bis 30 Aminosäuren besteht, wobei, das Peptid durch rechnergestützten Entwurf oder aus einer Datenbank für synthetische Peptide unter Verwendung einer Zielzelle oder eines Zielmoleküls ausgewählt wurde.

2. Das Teilchen nach Anspruch 1, **dadurch gekennzeichnet, dass** sein Durchmesser 10 nm oder weniger ist.

3. Des Teilchen nach Anspruch 1 oder 2, **dadurch gekennzeichnet, dass** mindestens einer der Konstituenten, die in den Kern eingeschlossen sind, aus mindestens einem Metall oder einem Derivat davon wie einem Oxid besteht.

4. Das Teilchen nach einem der Ansprüche 1 bis 3, **dadurch gekennzeichnet, dass** der Kern und die Beschichtung keine getrennten Einheiten bilden, sondern eine chemisch homogene Masse aufbauen.

5. Das Teilchen nach einem der Ansprüche 1 bis 4 zur Verwendung als therapeutisches Mittet in Kombination mit einem alternierenden homogenen Magnetfeld oder Gradienten-Magnetfeld zur Behandlung verschiedener Arten von Krankheiten.

6. Das Teilchen nach Anspruch 5, welches dazu vorgesehen ist, Krebs, Pilz- und bakterielle Infektionen, Protozoen-Infektionen, Virus-Infektionen, Stoffwechselkrankheiten, Immunschwächekrankheiten und genetisch begründete Krankheiten zu behandeln.

7. Das Teilchen nach Anspruch 5 oder 6, **dadurch gekennzeichnet, dass** der therapeutische Effekt des Teilchens durch Modifizieren seiner Oberfläche mit einem oder mehreren Effektormolekülen weiter unterstützt wird, welche unter anderem Zytostatika, Toxine, Enzyme, Schwermetalle, die Biokompatibilität unterstützende Substanzen und Substanzen, welche den Transport des Teilchens durch Gefäßwände und Gefäßgewebe erleichtern, umfassen.

8. Das Teilchen nach einem der Ansprüche 1 bis 4 zur Verwendung als Subkomponente in einem biologischem Assay.

## Revendications

1. Particule à réponse magnétique, **caractérisée en ce qu'**elle comprend un coeur super paramagnétique et un revêtement externe qui contient au moins un type de peptide non natif synthétique avec au moins un élément de reconnaissance, constitué de 1 à 30 acides aminés, dans laquelle ledit peptide a été choisi par conception informatique ou à partir d'une bibliothèque de peptides synthétiques en utilisant une cellule cible ou une molécule cible.

2. Particule selon la revendication 1, **caractérisée en ce que** son diamètre est de 10 nm ou moins.

3. Particule selon la revendication 1 ou 2, **caractérisée en ce qu'**au moins un des constituants compris dans le coeur est constitue d'au moins un métal ou un dérivé de celui-ci, tel qu'un oxyde.

4. Particule selon l'une quelconque des revendications 1 à 3, **caractérisée en ce que** le coeur et le revêtement ne forment pas d'unité séparée mais constituent une masse chimiquement homogène.

5. Particule selon l'une quelconque des revendications 1 à 4 pour une utilisation en tant qu'agent thérapeutique en combinaison avec un champ magnétique homogène alternatif ou un gradient de champ magnétique pour traiter différents types de maladies.

6. Particule selon la revendication 5 destinée à traiter un cancer, des infections fongiques et bactériennes, des infections protozoaires, des infections virales, des troubles métaboliques, des maladies des défenses immunitaires et des maladies génétiquement en rapport.

7. Particule selon la revendication 5 ou 6, **caractérisée en ce que** l'effet thérapeutique de la particule a été en outre stimulé par modification de sa surface avec une ou plusieurs molécules effectrices comprenant, entre autres, des cytostatiques, des toxines, des enzymes, des métaux lourds, des substances de promotion de la biocompatibilité, des substances qui facilitent le transport de la particule au travers des parois et des tissus vasculaires.

8. Particule selon l'une quelconque des revendications 1 à 4, pour une utilisation en tant que sous-composant dans un dosage biologique.
